**Europäisches Patentamt**

**European Patent Office**

**Office européen des brevets**

(19)

(11) Veröffentlichungsnummer: **0 153 471**
**B1**

(12) **EUROPÄISCHE PATENTSCHRIFT**

(45) Veröffentlichungstag der Patentschrift:
16.09.87

(51) Int. Cl.⁴: **A 23 K 1/16**

(21) Anmeldenummer: **84115327.3**

(22) Anmeldetag: **13.12.84**

(54) **Verwendung von Komplexverbindungen aus Inosin bei der Mast von Nutztieren.**

(30) Priorität: **08.02.84 DE 3404315**

(43) Veröffentlichungstag der Anmeldung:
**04.09.85 Patentblatt 85/36**

(45) Bekanntmachung des Hinweises auf die Patenterteilung:
**16.09.87 Patentblatt 87/38**

(84) Benannte Vertragsstaaten:
**AT BE CH DE FR GB IT LI LU NL SE**

(56) Entgegenhaltungen:
**CH - A - 460 501**
**CH - A - 467 025**
**DE - A - 1 692 496**

(73) Patentinhaber: **Newport AG, Alpenstrasse 2,
CH-6300 Zug (CH)**

(72) Erfinder: **Ginsberg, Theodore, Dr., 3009 Dorn Court,
Laguna Beach California 92651 (US)**
Erfinder: **Lira, Miguel, Romero, Dr., Tlapancalco 14 i,
Delegacopm Cpupacan 04000 Mexico 21 (US)**

(74) Vertreter: **Fiedler, Otto Karl, Dipl.-Ing., Patentanwalt
Hug Interlizenz AG Austrasse 44 Postfach,
CH-8045 Zürich (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Verwendung einer Komplexverbindung aus Inosin und einem Säureadditionssalz von Dimethylaminoisopropanol oder einer Mischung aus diesen Komponenten zur Wachstumsförderung und Futtereinsparung bei der Intensivmast von domestizierten Nutztieren wie von Schweinen, Geflügel, Rindern, Pferden, Schafen, Hunden und Fischen geeignet ist.

Angesichts des ständig steigenden Bedarfes an billigem tierischem Eiweiss durch die sich rasch vermehrende Erdbevölkerung begleitet von einer stagnierenden oder sich sogar vermindernden landwirtschaftlich nutzbaren Landfläche muss aus einer gegebenen Futtermenge immer mehr Fleisch erzeugt werden.

Es ist daher notwendig, die Produktion von ernährungsphysiologisch wertvollem Schweine-, Geflügel-, Rind-, Pferde-, Schaf- und Fischfleisch durch den Einsatz von wachstumsfördernd und futtersparend wirkenden Zusätzen zum Futter dieser Nutztiere zu rationalisieren.

Als wachstumsfördernde Mittel wurden bisher Antibiotika mit schmalem Wirkungsspektrum wie beispielsweise Zinkbacitracin, Penicillin, Streptomycin aber auch Antibiotika mit breitem Wirkungsspektrum wie Tetracyclin, Oxytetracyclin oder Chlortetracyclin in grossem Ausmasse verwendet. Diese Mittel wirken gewöhnlich indirekt wachstumsfördernd einerseits durch Hemmung bzw. Unterdrückung bestimmter Keime im Magen-Darmkanal, womit der sonst für die Erhaltung dieser Keime verwendete Futteranteil vom Tier nun auch zur Fleischerzeugung herangezogen wird und andererseits durch Verhütung von energieabsorbierenden Infektionskrankheiten.

Die Verwendung von Antibiotika als wachstumsfördernde Zusätze in Futtermitteln ist humanmedizinisch nicht unbedenklich. Es besteht die Gefahr, dass durch die anhaltende, hochdosierte Verfütterung von Antibiotika Fleisch produziert wird, das Spuren von Antibiotika enthält, die zu Resistenzbildungen beim Fleischkonsumenten Anlass geben können. Die Warnungen von seiten der Wissenschaft haben bereits zu behördlichen Beschränkungen geführt. Es ist damit zu rechnen, dass die regelmässige, nicht therapeutischen Zwecken dienende Verwendung von Antibiotika im Tierfutter verboten wird.

Ausser Antibiotika wurden jedoch auch andere, in der Humanmedizin verwendete stark antimikrobiell wirkende Chemotherapeutika als wachstumsfördernde Zusatzfuttermittel verwendet, beispielsweise Furazolidon [3-(5-Nitro-2-furfurylidenamino)--oxazolidon-(2)] vergl. z.B. Jucker et al, Wiener Tierärztliche Monatsschrift 60.213. 100-103, oder Sulfanilamide, vergl. z.B. Schweizer Patentschrift Nr. 467 025.

Furazolidon hat den Nachteil einer verhältnismässig starken Toxizität. Die prophylaktische Verwendung von Sulfanilamiden ist heute verboten.

Es kommen neuerdings immer mehr antimikrobiell wirkende Chemotherapeutika als wachstumsfördernde Zusatzfuttermittel in Gebrauch, die speziell für diesen Zweck entwickelt wurden. Dazu gehören das unter dem Gattungsnamen (Generic name) Cardabox bekanntgewordene 2-(2-Chinoxalinylmethylen)hydrazincarbonsäuremethylester-$N^1$,$N^4$-dioxid und das Olaquindoc 2-(N-2'-Hydroxyethylcarbamoyl)-3-methylchinoxalin-di-N-oxid. Beide Verbindungen sind verhältnismässig toxisch. Gegen deren Verwendung sind ausserdem Bedenken hinsichtlich einer möglichen cancerogenen Wirkung erhoben worden.

Neuerdings wird auch das unter der allgemeinen bezeichnung (Generic name) Nitrovin bekanntgewordene 1,5-Bis-(5-nitro-2-furyl)-1,4-pentadien-3--on-amidinhydrazon-hydrochlorid — Schweizer Patentschrift Nr. 460 501; Schneider et al, Landwirtschaftliche Forschung 1970.23(4), S. 350-352 — als wachstumsfördernder Zusatz zu Futtermitteln empfohlen. Auch in der Humanmedizin nicht verwendete Antibiotika wie beispielsweise Virginiamycin werden als wachstumsfördernde Wirkstoffe dem Futter zugesetzt. Diese Mittel führen in der Regel über die Unterdrückung der Darmflora und damit deren futterverzehrendem Stoffwechsel zu einer Wachstumsförderung. Gleichzeitig werden infektionsbedingte, die Futterverwertung senkende Durchfälle, Salmonellosen, Koli-Enteritiden und dergleichen verhütet.

Auch gegen die Verwendung dieser neuen Chemotherapeutika während der ganzen Dauer der Aufzucht sind Bedenken wach geworden. Das so gewonnene Fleisch enthält Spuren dieser Chemotherapeutika, die hygienisch nicht ganz unbedenklich sind. Es können sich Kreuzresistenzen bilden. Antimikrobielle Hemmstoffrückstände verursachen darüber hinaus bei der mikrobiellen Verarbeitung tierischer Produkte technologische Probleme (Eichhoff, MERCK-Kontakte 3/74 Seite 34.)

Ähnlich wie bei den Pestiziden sind konzentriertere Ablagerungen dieser körperfremden, toxischen Stoffe in bestimmten Organen prinzipiell möglich. Eine zurückhaltende und sparsame Anwendung ist deshalb angezeigt.

In der DE-A 1 692 496 wurde auch vorgeschlagen, 5'-Nucleotide wie Cytidin-, Adenosin-, Guanosin-, Uridin- und Inosin-5'-monophosphat für die Wachstumsbeschleunigung bei der Fütterung von Nutztieren zu verwenden. Diese Phosphorsäureester haben den Nachteil, dass sie teuer und verhältnismässig unbeständig sind.

Es besteht demnach ein eminentes Bedürfnis an preiswerten wachstumsfördernden und futtersparenden Wirkstoffen, welche keine direkten chemotherapeutischen Wirkungen entfalten, toxikologisch vollkommen unbedenklich sind und insbesondere keine unnatürlichen oder unerwünschten Rückstände im Fleisch hinterlassen.

Der Erfindung liegt die Aufgabe zugrunde, einen Wirkstoff zu finden, der diesen hohen Anforderungen genügt und wirtschaftlich tragbar ist.

Es wurde gefunden, dass die Komplexverbindung aus Inosin und einem Säureadditionssalz von Dimethylaminoisopropanol oder eine Mischung aus diesen Komponeten die gewünschte wachstumsfördernde Wirkung bei der Mast von Nutztieren entfaltet, ohne dass damit irgendwelche physiologischen Nachteile in Kauf genommen werden müssen.

Die durch Verabreichung der Inosin-Komplexverbindung bei der Mast von Nutztieren erzielte Gewichtszunahme liegt bereits nach einigen Wochen etwa 10% über der Norm.

Komplexverbindungen aus Inosin und Säureadditionssalzen von Dimethylaminoisopropanol, insbesondere aus Inosin und Dimethylaminoisopropanol·4-acetaminobenzoat, sind aus der Patentliteratur bekannt. Sie sind als nicht spezifische Mittel mit breitem Wirkungsspektrum gegenüber den verschiedensten DNS- und RNS-Viren sowie als Mittel zur Verbesserung des Lernvermögens und als Härter für Melamin und Harnstofformaldehydharze beschrieben worden.

Untersuchungen bei einer Vielzahl von Viruserkrankungen ergaben, dass diese Inosin-Komplexverbindungen nicht direkt chemotherapeutisch wirken. Sie stimulieren vielmehr das natürliche Immunsystem, erhöhen die Immunreaktion der Wirtzellen und hemmen damit die Reproduktion von Virusproteinen.

Dieser Wirkungsmechanismus und die Zusammensetzung der Komplexverbindung aus Inosin und Dimethylaminoisopropanol·4-acetaminobenzoat garantieren die absolute Unschädlichkeit dieses Wirkstoffes auch bei Dauerverwendung hoher Dosen. Inosin (6-Hydroxypurin-9D-ribofuranosid) ist ein natürlicher Bestandteil im Fleisch und kommt auch in Hefen, Zuckerrüben und vielen weiteren Nahrungsmitteln vor. Inosin aktiviert die Zellfunktionen. 4-Aminobenzoesäure ist identisch mit dem Wuchsstoff H, der aus Hefen isoliert wurde und beispielsweise von Bakterien in den für Einzeller lebenswichtigen Wuchsstoff Folsäure eingebaut wird. 4-Aminobenzoesäure wird aus dem Organismus von Wirbeltieren vorwiegend in Form von 4-Acetaminobenzoesäure ausgeschieden. Es kann daher nicht verwundern, dass Dimethylaminoisopropanol·4-acetaminobenzoat ausserordentlich gut verträglich ist und überdies rasch aus dem Organismus der Wirbeltiere ausgeschieden wird. Rückstände, die vom Wirkstoff stammen und die nicht ohnehin im Organismus von Nutztieren natürlich vorkommen, konnten daher verständlicherweise nie nachgewiesen werden.

Damit ist die Inosin-Komplexverbindung allen bisher verwendeten wachstumsfördernd wirkenden Mitteln bezüglich Veträglichkeit bei weitem überlegen. Aus gleichen Gründen ist das mit dessen Mithilfe erzeugte Fleisch für den menschlichen Verzehr völlig unbedenklich, was von bisherigen Mitteln nie mit Sicherheit nachgewiesen werden konnte.

Der wachstumsfördernde bzw. futtereinsparende Effekt der Inosin-Komplexverbindung ist nicht auf einzelne Nutztierarten beschränkt. Vergleichende Mastversuche mit frisch geworfenen Ferkeln und mit Jagern (Schweinen von ca. 40 kg Gewicht) sowie mit Hühner- und Trutenküken, mit Kälbern, Fohlen, Lämmern, Hundewelpen sowie Karpfen und Forellen haben erwiesen, dass durch Verabreichung der Komplexverbindung aus Inosin und Dimethylaminoisopropanol·4-acetaminobenzoat zusätzlich zum normalen Futter in allen Fällen eine höhere Gewichtszunahme resultiert als bei den Kontrolltiergruppen, die neben derselben Futtermenge nur Placebo erhielten.

Dieselben Resultate wurden erzielt, wenn anstelle der Inosin-Komplexverbindung eine Mischung aus Inosin und Dimethylaminoisopropanol·4-acetaminobenzoat verabreicht wurde, vorausgesetzt, dass die Mischung beide Komponenten in ausreichender Menge enthielt.

Bei den Mastversuchen konnte auf die Verwendung von Antibiotika verzichtet werden. Trotzdem waren auch unter nur durchschnittlichen hygienischen Bedingungen, unter denen Infektionen nicht auszuschliessen sind, die Tiere im allgemeinen während der ganzen Periode der Verabreichung der Inosin-Komplexverbindung bei augezeichneter Gesundheit.

Die Versuchstiere, welche die Insosin-Komplexverbindung erhielten, waren im allgemeinen munterer und vitaler als die Kontrolltiere.

Gegenstand der Erfindung ist die Verwendung einer Komplexverbindung aus inosin und einem Säureadditionssalz von Dimethylaminoisopropanol oder eine Mischung aus diesen Komponenten zur Wachstumsförderung und Futtereinsparung bei der Intensivmast von domestizierten Nutztieren, insbesondere bei der Mast von Schwein, Geflügel, Rindern, Pferden, Schafen und Fischen.

Als Säuren für die Bildung der Säureadditionssalze von Dimethylaminoisopropanol eignen sich im Prinzip alle Säuren, die gut verträgliche Salze bilden wie zum Beispiel Salzsäure, Phosphorsäure, Acetylsalizylsäure, 4-Aminobenzoesäure usw. Der Komplex aus Inosin und Dimethylaminoisopropanol·4-acetaminobenzoat wird im allgemeinen bevorzugt, da er physiologisch besonders geeignet und zudem gegenüber Feuchtigkeit stabiler ist als die Komplexsalze der übrigen genannten Säuren. Gewöhnlich wird die Komplexverbindung aus 1 Mol Inosin und 3 Mol Dimethylaminoisopropanol·4-acetaminobenzoat oder eine Mischung dieser Komponenten im angegebenen Molverhältnis benutzt.

Dieser Wirkstoff eignet sich zur Mast von Schweinen, insbesondere zur Aufzucht von frisch geborenen Ferkeln aber auch für die Intensivmast von Jagern.

Derselbe Wirkstoff kann aber auch zur Wachstumsförderung bei der Mst von Hühnerküken, Broilern, Truthühnern, Enten und Gänsen verwendet werden.

Die Inosin-Komplexverbindung dient auch zur beschleunigten Aufzucht und Futtereinsparung bei Kälbern und bei der Mast von Ochsen. Gute Erfolge wurden bei der Verwendung dieses Wirkstoffes bei der Intensivmast von Lämmern erzielt.

Schliesslich kann man den Wirkstoff zur Futtereinsparung bei der Aufzucht von Fischen wie z.B. Karpfen, Forellen, Aeschen, Felchen, Hechten, Zander, Grasfischen, Welsen, Aalen in Teichhaltungen und sogenannten Aquakulturen verwenden.

Der Wirkstoff wird, seinem Verwendungszweck entsprechend, den Tieren in der Regel oral verabreicht.

Bei der Bereitung der individuellen Darreichungsformen müssen die Besonderheiten der betreffenden Tierart sowie deren Alter berücksichtigt werden. Es muss dafür gesorgt werden, dass jedes Tier seine bestimmte dosierte Menge Wirkstoff auch wirklich erhält und keine vermeidbaren Verluste durch Verschütten oder Beschmutzen eintreten. Jungtieren, die noch bei der Mutter saugen, kann der Wirkstoff zur optimalen Kontrolle in Form einer wässerigen Lösung direkt in den Schlund gespritzt werden. Bei Kälbern kann der Wirkstoff aufgelöst in Milch verfüttert werden. Eine Verabreichung ist auch im Trinkwasser möglich. Tieren, die bereits feste Nahrung einnehmen, kann der Wirkstoff dem Futter, insbesondere dem Kraftfutter zugesetzt werden. Gut bewährt hat sich die Verabreichung in Pellets oder Würfeln, die ausser dem Wirkstoff noch Nahrungsbestandteile enthalten, welche die betreffenden Tiere besonders schätzen wie z.B. Melasse, Fette, pflanzliche oder tierische Proteine (Sojabohnenschrot, Getreideschrot, Hefepulver, Molke, Casein, Algenpulver, Fischmehl usw.), Vitamine, Salze und Spurenelemente.

Fischen wird der Wirkstoff in Kapseln von etwa 1 bis 7 mm Durchmesser verabreicht, die sich im Wasser bei Raumtemperatur nicht auflösen. Zweckmässig ist auch die Verabreichung in Fette enthaltenden Futterpellets aus denen der Wirkstoff durch Wasser nicht oder kaum herausgelöst wird.

Dosierung des Wirkstoffes

Der Wirkstoff besteht in der Regel aus der Komplexverbindung aus 1 Mol Inosin und 3 Mol Dimethylaminoisopropanol · 4-acetaminobenzoat. Kommt als Komplexbilder für das Inosin ein Säureadditionssalz einer andern Säure zur Anwendung, so ändert sich die Dosierung entsprechend der Änderung des Gesamtmolekulargewichtes.

Die Dosierungen sind der Tierat, dem Alter der Tiere und den Besonderheiten der Tierhaltung anzupassen. Generelle Aussagen haben daher für den Einzelfall nur limitierte Bedeutung. Im allgemeinen ist es zweckmässig, mit der Wirkstoffgabe kurz nach der Geburt bzw. dem Schlüpfen zu beginnen, um von Anfang an das Immunabwehrsystem der Tiere zu stimulieren.

Junge Schweine erhalten in den ersten Wochen täglich etwa 60 bis 80 mg Wirkstoff pro Tier. Die Dosierung wird gewöhnlich in den ersten 6 Wochen bei 40 bis 80 mg pro kg Körpergewicht gehalten.

Hühnerküken erhalten den Wirkstoff in Pellets, wobei die Menge des Wirkstoffes etwa 20 bis 100 mg pro kg Gesamtfuttermenge (ohne Trinkwasser) beträgt.

Broiler erhalten 50 bis 500 mg Wirkstoff pro kg Gesamtfuttermenge.

Kälber, die bei der Mutter saugen dürfen, benötigen zunächst verhältnismässig wenig Wirkstoff, da sie Abwehrstoffe mit der Muttermilch erhalten. In dieser Phase werden an 5 von 7 Tagen jeweils etwa 1 bis 10 g Wirkstoff verabreicht.

Kälber, die eine aus den erforderlichen Nahrungskomponenten künstlich zusammengesetzte Milch erhalten, wird der Wirkstoff dieser Milch in Dosen von 50 bis 500 pro kg Körpergewicht und Tag zugesetzt. Kälbern und jungen Ochsen wird der Wirkstoff in Dosen von 100 bis 1000 mg/kg Körpergewicht und Tag dem Kraftfutter zugesetzt, wobei der Wirkstoff dem Kraftfutter in Form einer Vormischung (Premix), aus Sojamehl enthaltend 20 bis 50 % Wirkstoff, zugesetzt wird.

Fohlen, Lämmern und Welpen, die in der Regel bei der Mutter saugen dürfen, wird der Wirkstoff ähnlich wie bei saugenden Kälbern und in ähnlichen Dosen verabreicht.

Fische erhalten, sobald sie feste Nahrung aufnehmen können, etwa 50 bis 500 mg Wirkstoff pro kg Futter.

Fische im Alter von 3 Monaten bis 1 Jahr erhalten 100 bis 1000 mg Wirkstoff pro kg Futter.

*Beispiele*

*Beispiel 1*

Je 5 Würfe von 8 jungen 5 Tage alten Ferkeln erhielten während 6 Wochen zusätzlich zum normalen Futter an 5 von 7 Tagen pro Woche jeweils 50 mg Wirkstoff aus 1 Mol Inosin und 3 Mol Dimethylaminoisopropanol · 4-acetaminobenzoat pro kg Körpergewicht gelöst in 25 ml Wasser oder dasselbe Volumen Placebo (Wasser dem 2 % Kochsalz zugesetzt wurde) direkt in den Schlund gespritzt.

Nach 6 Wochen waren die Würfe, welche den Wirkstoff erhielten, jeweils zusammen 2,5 bis 3 kg schwerer als die Kontrollwürfe. Der Gewichtszuwachs lag + 10% über der Norm.

*Beispiel 2*

Schweinemastvergleichsversuch (Jagermast)

Eine Kollektion von 20 Jagern (Schweinen von ca. 40 kg Gewicht) wurde in Einzelbuchten mit einem Basisfutter folgender Zusammensetzung gemästet:

| | | |
|---|---|---|
| 17 | % | Rohprotein |
| 14 | % | verdauliches Rohprotein |
| 5 | % | Rohfaser |
| 66 | % | Stärkeeinheiten |
| 0,7% | | Lysin |
| 1,0% | | Premix (aus 50% Wirkstoff enthaltend 1 Mol Inosin und 3 Mol Dimethylaminoisopropanol · 4-acetaminobenzoat und 50% Sojamehl) |
| oder | 1,0% | Placebo aus 100% Sojamehl |

Nach 53 Tagen wurden die Tiere gewogen.

Die mit dem Wirkstoff behandelten Tiere zeigten im Mittel eine um + 9,5% höhere Gewichtszunahme als die Kontrolltiere.

*Beispiel 3*

Hühnermast

Durch Zusatz von 500 ppm Wirkstoff aus 1 Mol Inosin + 3 Mol Dimethylaminoisopropanol · 4-acetaminobenzoat zum Futter von Hühnern lassen sich folgende Ergebnisse erzielen:

| | |
|---|---|
| Mastküken | Gewichtszunahme gegenüber der Norm + 5-7% Verbesserung der Futterverwertung (kg Futter/kg Zuwachs) + 4,5% |
| Broilermast | Gewichtszunahme + 12-15% über der Norm |
| (Mastdauer | Futterverwertung + 12-13%. 50 Tage). |

*Beispiel 4*

Kälbermastvergleichsversuch

Je 20 entwöhnte Kälber erhielten eine künstlich zusammengesetzte Milch, die alle notwendigen Nahrungsbestandteile in ortsüblichen Dosierungen enthielt. Der Hälfte der Kälber wurden an 6 von 7 Wochentagen jeweils 6 g Wirkstoff aus 1 Mol Inosin und 3 Mol Dimethylaminoisopropanol·4-acetaminobenzoat, aufgelöst in der «Milch», verabreicht.

Nach 50 Tagen wurden die Tiere gewogen.

Die Tiere, welche den Wirkstoff erhielten, zeigten im Mitteln einen um +7,3% höheren Gewichtszuwachs als die Kontrolltiere.

*Beispiel 5*

Ochsenmastversuch

24 Ochsen mit einem durchschnittlichen Einstellgewicht von 100 kg wurden in 2 Gruppen aufgeteilt und gleichmässig mit Kraftfutter, das alle erforderlichen Zusätze und Spurenelemente sowie zusätzlich mit Maissilage, Maiskolbenschrot und Heu gemästet.

Dem Kraftfutter von 12 Tieren wurde jeweils 550 ppm Wirkstoff aus 1 Mol Inosin und 3 Mol Dimethylaminoisopropanol·4-acetaminobenzoat zugesetzt.

Nach 105 Tagen war die Gewichtszunahme der Tiere, die den Wirkstoff erhielten, signifikant (+8%) höher als die der Kontrollen.

*Beispiel 6*

Lämmermast

Lämmer wurden mit Kraftfutter, welches ausreichende Mengen von Spurenelementen enthielt, und mit Heu gefüttert. Die Hälfte der Tiere erhielt mit dem Kraftfutter 1000 ppm Wirkstoff aus 1 Mol Inosin +3 Mol Dimethylaminoisopropanol·4-acetaminobenzoat.

Die Gewichtszunahme der Lämmer, welche den Wirkstoff erhielten, war +10% höher als die der Kontrolltiere.

*Beispiel 7*

Fischmast

Spiegelkarpfen wurden mit normalem pelletiertem Futter, das 1000 ppm Wirkstoff aus 1 Mol Inosin +3 Mol Dimethylaminoisopropanol·4-acetaminobenzoat enthielt, gemästet. Diese Massnahme bewirkte eine signifikant erhöhte Gewichtszunahme gegenüber normal ernährten Karpfen.

Ähnliche Effekte werden auch bei Forellen erzielt. Dabei fällt auf, dass die Ausfallrate der Fische, die den Wirkstoff erhielten, beträchtlich niedriger ist als bei den Tieren, die nur das übliche Futter bekamen.

## Patentansprüche

1. Verwendung einer Komplexverbindung aus Inosin und einem Säureadditionssalz von Dimethylaminoisopropanol oder einer Mischung aus diesen Komponenten als Wirkstoff zur Wachstumsförderung und Futtereinsparung bei der Mast von Schweinen, Geflügel, Rindern, Pferden, Schafen, Hunden und Fischen.

2. Verwendung nach Patentanspruch 1 einer Komplexverbindung aus Inosin und Dimethylaminoisopropanol·4-acetaminobenzoat oder einer Mischung aus diesen Komponenten als Wirkstoff.

3. Verwendung nach Patentanspruch 2 der Komplexverbindung aus 1 Mol Inosin und 3 Mol Dimethylaminoisopropanol·4-acetaminobenzoat oder einer Mischung dieser Komponenten im angegebenen Molverhältnis als Wirkstoff.

4. Verwendung des Wirkstoffes nach Patentanspruch 3 bei der Mast von Schweinen.

5. Verwendung des Wirkstoffes nach Patentanspruch 3 bei der Mast von Geflügel.

6. Verwendung des Wirkstoffes nach Patentanspruch 3 bei der Mast von Rindern.

7. Verwendung des Wirkstoffes nach Patentanspruch 3 bei der Mast von Schafen.

8. Verwendung des Wirkstoffes nach Patentanspruch 3 bei der Mast von Fischen.

## Claims

1. Use of a complex of inosine and an acid addition salt of dimethylaminoisopropanol or a mixture of these components as an active compound for promoting growth and saving feed in the fattening of pigs, poultry, cattle, horses, sheep, dogs and fish.

2. Use, according to patent claim 1, of a complex of inosine and dimethylaminoisopropanol 4-acetaminobenzoate or a mixture of these components as an active compound.

3. Use, according to patent claim 2, of the complex of 1 mol of inosine and 3 mol of dimethylaminoisopropanol 4-acetaminobenzoate or a mixture of these components in the stated molar ratio as an active compound.

4. Use of the active compound according to patent claim 3 for the fattening of pigs.

5. Use of the active compound according to patent claim 3 for the fattening of poultry.

6. Use of the active compound according to patent claims 3 for the fattening of cattle.

7. Use of the active compound according to patent claim 3 for the fattening of sheep.

8. Use of the active compound according to patent claim 3 for the fattening of fish.

## Revendications

1. Utilisation d'un complexe d'inosine et d'un sel d'addition acide du diméthylaminoisopropanol ou d'un mélange de ces composants, comme matière active pour favoriser la croissance et économiser la nourriture dans l'élevage des porcs, volailles, boeufs, chevaux, moutons, chiens et poissons.

2. Utilisation selon la revendication 1 d'un complexe d'inosine et de diméthylaminoisopropanol·4-acétaminobenzoate ou d'un mélange de ces composants comme matière active.

3. Utilisation selon la revendication 2 du complexe formé par 1 mole d'inosine et 3 moles de diméthylaminoisopropanol·4-acétaminobenzoate ou d'un mélange de ces composants dans le rapport molaire indiqué, comme matière active.

4. Utilisation de la matière active selon la revendication 3 dans l'élevage des porcs.

5. Utilisation de la matière active selon la revendication 3 dans l'élevage de volailles.

6. Utilisation de la matière active selon la revendication 3 dans l'élevage des boeufs.

7. Utilisation de la matière active selon la revendication 3 dans l'élevage des moutons.

8. Utilisation de la matière active selon la revendication 3 dans l'élevage des poissons.